# EUROPEAN PATENT SPECIFICATION

(11) **EP 2 629 726 B1**
(45) Date of publication and mention of the grant of the patent: **14.10.2015**
(21) Application number: 11724474.9
(22) Date of filing: 23.03.2011
(51) Int. Cl.: A61F 13/15, A61F 13/20, A61F 13/22

(54) **HYGIENIC TAMPON WITH IMPROVED SLIDING ABILITY AND METHOD FOR MANUFACTURING THEREOF**
TAMPON FÜR KÖRPERHYGIENE MIT VERBESSERTER GLEITFÄHIGKEIT UND HERSTELLUNGSVERFAHREN DAFÜR
TAMPON HYGIÉNIQUE AVEC CAPACITÉ DE COULISSEMENT AMÉLIORÉE ET PROCÉDÉ POUR LA FABRICATION DE CELUI-CI

(30) Priority: 02.03.2011 SI 201100068; 20.10.2010 SI 201000333
(43) Date of publication of application: 28.08.2013
(73) Proprietor: TOSAMA Tovarna sanitetnega materiala d.o.o., 1230 Domzale (SI)
(72) Inventor: ZABRET, Andrej, 1218 Komenda (SI)
(74) Representative: Borstar, Dusan
(86) International application number: PCT/SI2011/000015
(87) International publication number: WO 2012/053986

(56) References cited:
- EP-A1- 0 611 562
- WO-A1-99/27878
- WO-A2-02/076357
- AU-B2- 643 768
- US-A- 5 813 102
- US-A1- 2003 135 180

## Description

The present invention refers to a tampon, in particular to a hygienic tampon. In accordance to the International patent classification, such inventions belong to human necessities, namely to hygiene, and in particular to catamenial tampons and accessories therefor.

It is an object of the invention, how to improve the sliding ability on the outer surface of a tampon equipped with ribs and grooves, by which such tampon would however still have to be manufactured of the same raw materials, by using the same manufacturing equipment and by performing essentially the same processing steps like in manufacturing any other modern and seriously commercialized tampon, and by which the other essential properties of such tampon should not be hindered, which in particular refers to maintaining its absorbency for liquids including a specific absorption capacity and the rate of absorption, preventing of leakage as well as maintaining of buckling strength and stability of dimensions of such tampon.

A tampon of the prior art together with apparatus and process for manufacturing thereof is disclosed in EP 0 611 562 A1.

A tampon, which is described in EP 0 422 660, consists of a nonwoven fibrous blank, which is obtained by means of winding of a nonwoven fibrous strip around its transversal axis, by which appropriately cylindrical blank is formed, which is then radially compressed by means of corresponding jaws e.g. in a corresponding press according to DE 19 825 877 into a shape of a tampon. Such tampon is approximately cylindrical, although radially inwards directed grooves are pressed on its surface, which extend in the axial direction i.e. parallel to said longitudinal axis of the tampon. Such tampons are generally known to those skilled in the art as so called "digital tampons". A rib is available between each two neighboring grooves, wherein the diameter of such tampon is determined by the circumferential portions of said ribs, while the bottom portions of said groove determine a highly compressed core on the tampon. In a subsequent step of manufacturing such tampon is slightly compressed in the area of said ribs, by means of which then the lateral portions of ribs become convex, upon which the lateral flanks of each neighboring ribs shall contact each other, so that closely to said core a tubular and parallel with the longitudinal axis of the tampon extending cavity is formed between each pair of ribs. Such concept should lead to certain advantages, e.g. to increasing of buckling strength and in particular to increasing of specific absorption capacity and the absorption rate in the longitudinal direction of the tampon. Said buckling strength is important by inserting the tampon and has some influence on perception of consumers in view of reliability. In addition to said buckling strength it is however also very important by inserting the tampon that the outer surface thereof, which is during the use of such tampon in contact with mucosa, shall be as smooth as possible, so that the coefficient of friction on said outer surface should be the lowest possible.

As proposed in EP 1 014 910, a smooth outer surface may be achieved during compression of a fibrous material into a shape of tampon, by which the pressure needs to be essentially higher than in compression of previously known tampons. Thanks to such extremely high pressure, stability of the shape of the tampon should be essentially improved, and at the same time the outer surface of the tampon should also be smooth. In accordance with said source the tampons may be manufactured, which are approximately cylindrical and are moreover without any ribs and groves on the outer surface, so that the specific absorption capacity of such tampons i.e. absorptiveness per quantity of fibers is then essentially lower than by tampons with ribs and grooves on the outer surface.

As described in US 4,981,884, stability of dimensions of a tampon may also be achieved by heating of pressing jaws, by means of which the fibrous material is formed into each tampon. Also in such case, at least approximately cylindrical tampon without any ribs and grooves on its outer surface may be manufactured, and the specific absorption capacity is then quite similar like by a previously mentioned tampon according to EP 1 014 910.

A further possible approach in view of providing a smooth outer surface of a tampon is described in WO 01/01910 A1, wherein the outer surface of such tampon is surrounded with a perforated covering layer on the basis of artificial fibers, which are as such unable to absorb the liquid, but the sliding ability is herewith be essentially improved. Due to the fact that said covering layer is perforated, the liquid is allowed to be transferred towards the interior of the tampon, where the liquid is then absorbed by appropriately absorptive natural fibers. The presence of said covering layer in a classic mass production process makes such process much more complicated, in particular when transporting throughout the machine is taken into consideration, and EP 1 244 402 has proposed the use of a non-ionic surfactant. As a consequence of such improving the sliding ability, the specific absorption capacity and in particular the absorption rate are then essentially reduced.

A still further approach in view of improvement of the sliding ability is proposed in EP 1 035 819 or US2003/0105444 A1, where during the step of winding natural fibrous material by forming an essentially cylindrical blank, of which then by means of radially compression by jaws a tampon is formed, a previously smoothed covering layer is attached onto said layer of natural fibers and is upon that wound together with said layer of natural fibers into said blank. A tampon is then formed by means of radial compression, which is along its complete circumference surrounded with a covering layer, wherein such tampon like e.g. a tampon according to EP 0 422 660 also comprises a plurality of ribs and grooves on its outer surface, so that in addition to satisfactory stability of the shape and buckling strength also acceptable absorption capacity and absorption rate are then achieved. Said smoothing of the covering layer having a two-component fibrous structure is performed by means of calendaring between appropriately heated rollers, by which the artificial fibers are plasticized and in such plasticized state also essentially, by which the liquid permeability of such smoothed covering layer is reduced in such extend that the covering layer becomes practically hydrophobic. As previously described, such smoothed covering layer is present along the complete circumference of such manufactured tampon, namely both on outer surfaces of the ribs, which during the use of the tampon stay in contact with mucosa, and also in the area of grooves between each pair of ribs.

Also the practical experience shows that thanks to such performed smoothing of the covering layer, the sliding ability on the outer surface of the tampon is definitively improved, but at the same time the process of absorption of the liquid into the tampon is also essentially changed due the presence of such at least partially hydrophobic covering layer in the area of grooves between the ribs. And because said smoothed and hydrophobic covering layer is also present between the ribs, the progress of the liquid in the axial direction of the tampon is much more intensive than the progress of the liquid in the radial direction towards the absorbent core, which should actually able to absorb an essential quantity of the liquid. It is therefore obvious that despite to expected storing the liquid such tampon shows tendency of transferring the liquid in its axial direction, which unavoidable leads to flowing out the liquid (which is known to those skilled in the art as the "leakage") on the opposite side of the tampon, which is absolutely undesired. And although the sliding ability would be still further improved e.g. by using a special fibrous structure of the covering layer according to EP 1 988 202 A1, by maintaining such covering layer around the complete circumferential area of the tampon and without essentially amending the concept of the tampon as such, the problem of preventing the leakage due to undesired process of absorption in view of tendency to transfer the liquid in axial instead of in radial direction would still remain completely unresolved.

The present invention refers to a hygienic tampon in accordance with claim 1 having improved sliding ability on its outer surface, wherein such tampon comprising an absorbent core consisting of absorbent natural or artificial fibers, as well as a of a continuous covering layer, which contains thermoplastic fibers which are approximately uniformly distributed across the surface thereof and by which said core is surrounded around its complete circumferential area, wherein the first end portion of such tampon is optionally narrowed and formed with a slightly rounded tip, while the second end portion thereof is equipped with a string intended for easier removing the tampon after the use, and wherein such tampon is due to at least approximately radial compression on its surface provided with appropriate number i.e. at least two to ten radial grooves, which extend at least approximately parallel to each other in the longitudinal direction of the tampon or are inclined at certain angle with respect to said longitudinal direction and which are equidistantly apart from each other distributed in the circumferential direction of the tampon, so that between each two neighboring grooves there is a rib having a slightly rounded convex outer surface, so that such outer surfaces of all ribs together form at least approximately cylindrical outer surface of the tampon.

In accordance with the invention and in order to assure improved sliding ability on its outer surface, such tampon is smoothed exclusively in the area of convex outer surfaces of each available ribs, by which on said outer surface of the tampon the friction coefficient is reduced and the sliding ability is correspondingly improved.

Consequently said covering layer of the tampon consists either of two-component fibers, where at least the outer component thereof consists of a thermoplastic material, or of a mixture of fibers, where at least a portion of these fibers represent thermoplastic fibers, and is at least in the area of convex outer surfaces of the ribs smoothed by heating said thermoplastic fibers or a thermoplastic component of said fibers up to the temperature of plasticizing of the thermoplastic material of fibers by simultaneously establishing a smooth uniform compression towards the outer surface of the covering layer in the radial direction regarding the tampon.

Said covering layer consists of two-component fibers, which are formed with a core on the basis of polyester (PES) and with a wrapper on the basis of high density polyethylene (PE) or optionally of a mixture of fibers of the same content, where the ratio PE : PES = 34% : 66% up to PE : PES = 50% : 50%, and where the specific surface density of such two-component fibers is 12 to 20 g/m² and preferably around 16 g/m², wherein said covering layer is smoothed in the area of convex outer surfaces of the ribs by means of plasticizing said polyethylene (PE) component of fibers at the temperature within the range between 120°C and 180°C, preferably between 122°C and 148°C, by simultaneously exposing such plasticized fibers on the outer surface of the covering layer (2) to a smooth uniform compression acting inwardly in the radial direction regarding the tampon.

A further object of the invention is a process of manufacturing in accordance with claim 2 a hygienic tampon having improved sliding ability on its outer surface, wherein such process comprising steps of
- preparing of appropriately thick layer of a non-woven fabric on the basis of natural or synthetic fibers for the purposes of forming an absorbent core;
- preparing of appropriately thick layer of a non-woven fabric for the purposes of forming a covering layer of the tampon, in which the content of fibers is determined in such manner that fibers are either two-component fibers in which one component consists of a thermoplastic material, or at least a portion of fibers are thermoplastic fibers;
- cutting said fabric into a strip intended to form said absorbent core and having a width adjusted to the length of the tampon and the length adjusted to each desired diameter of the tampon;
- cutting said fabric into a strip intended to form said covering layer and having a width adjusted to the length of the tampon and the length adjusted to each desired diameter or circumference of the tampon;
- assembling said strip intended to form the absorbent core and said strip intended to form the covering layer by means of overlapping said strip of the covering layer over a pre-determined area of the strip of the core, wherein this step also includes appropriate placing a string, which is intended for removing the tampon after the use thereof;
- winding said strip intended to form a core around the transversal axis of the strip, by which at least approximately cylindrical blank is formed, which is across the complete circumferential area or at least across the majority of said circumferential area surrounded by continuously extending strip intended to form the covering layer, wherein the diameter of such blank is adjusted by taking into consideration each expected diameter of the tampon, and wherein the length of such blank is adjusted with respect to each expected length of the tampon;
- introducing said blank into appropriate press in order to perform a radial compression thereof by means of appropriate jaws and to obtain at least cylindrical tampon having a narrowed and slightly rounded tip on its one end portion and a desired number, which corresponds to the number of jaws, of radial grooves, which are pressed in the circumferential surface of the tampon and extend at least essentially parallel to each other either in the longitudinal direction of the tampon or are inclined with respect to said longitudinal direction, and which are equidistantly spaced from each other in the circumferential direction of the tampon, so that a rib is formed between each two neighboring grooves, which is equipped with a slightly rounded convex outer surface, so that convex surfaces of all ribs form an essentially cylindrical outer surface of the tampon; and
- smoothing said outer surface of the tampon exclusively in the area of said convex outer surfaces of the ribs by means of pushing such formed tampon throughout a conical passage of a die in the axial direction thereof, by which the tampon is exposed to a slight compression acting radially inwards.

In accordance with the proposed invention, said step of smoothing the outer surface of the tampon is performed as smoothing of the outer surface of the covering layer consisting either at least partially of thermoplastic fibers, which are uniformly distributed across the surface area thereof, or of two-component fibers comprising outwardly located component on the basis of thermoplastic material, wherein exclusively in the area of said convex outer surfaces of the ribs on the tampon said thermoplastic fibers or a thermoplastic component of fibers are/is heated on the temperature of plasticizing of said thermoplastic fibers or said thermoplastic component of fibers, upon which such plasticized fibers are exposed to a slight and uniform compression, which is acting radially inwards with respect to the tampon.

The covering layer consisting of fibers having a core of polyester (PES) wrapped by high density polyethylene (PE) component, wherein the ratio PE : PES = 34% : 66% up to PE : PES = 50% : 50%, or optionally of two-component mixture of fibers of the same content of said materials, and wherein the surface density of such two-component fabric is 12 to 20 g/m² and preferably around 16 g/m², is smoothed in the area of convex outer surfaces of the ribs by means of heating said thermoplastic fibers up to the temperature of plasticizing said polyethylene (PE) fibers or a polyethylene (PE) component of said fibers by simultaneously exposing such plasticized fibers on the outer surface of the covering layer to a slight and uniform compression, which is acting inwardly in the radial direction regarding the tampon. For the purposes of said plasticizing of the polyethylene (PE) component, said fibers are heated up to the temperature within the range between 120°C and 180°C, preferably between 122°C and 148°C, upon which such plasticized fibers are exposed to a slight uniform compression, which is acting inwardly in the radial direction regarding the tampon.

The invention will be described on the basis of an embodiment, which is shown in the attached drawings, where
- Fig. 1: is a schematically shown cross-section of a tampon according to the invention;
- Fig. 2: schematically indicates the initial phase of manufacturing the tampon according to Fig. 1, namely winding the fibrous fabric together with a covering layer placed on, by which the blank is formed;
- Fig. 3: illustrates the final stage of manufacturing the tampon according to Fig. 1, namely compression of the blank according to Fig. 2 in the radial direction, which is then followed by smoothing of outer surfaces of ribs on such obtained tampon.

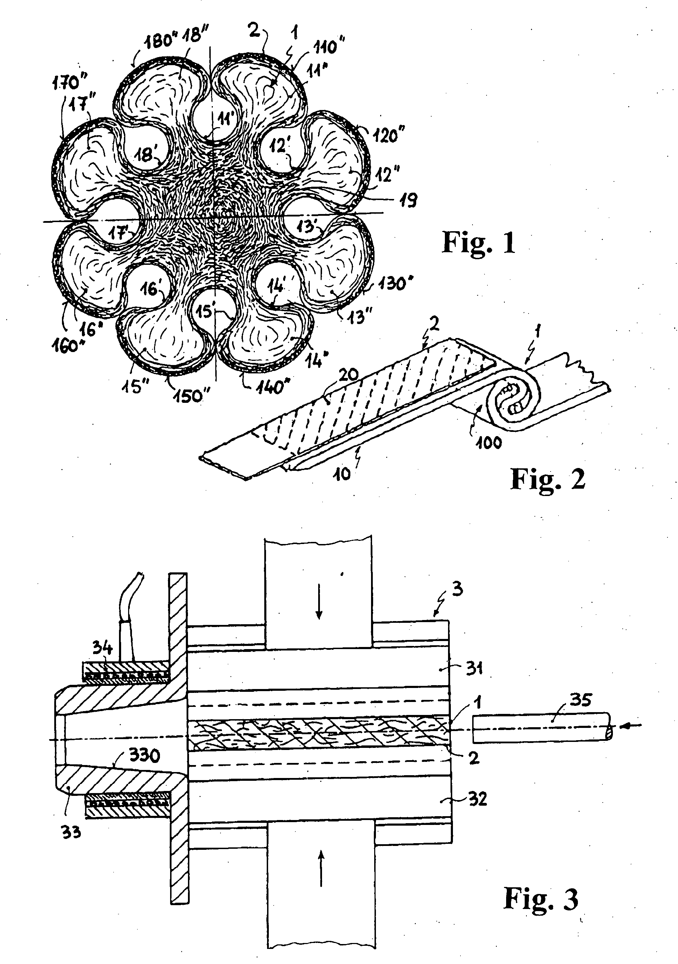

A tampon according to the invention (Fig. 1), which may either be a digital tampon adapted for inserting by means of fingers, or an applicator tampon adapted for inserting by means of appropriate requisite like a tampon applicator known to those skilled in the art, normally consist of an absorbent core 1, which is along its complete circumference surrounded with a covering layer 2, which should improve the sliding ability of the outer surface of the tampon.

Said absorbent core 1 of the tampon consists of a non-woven fabric on the basis of correspondingly absorbent natural and/or synthetic fibers, wherein e.g. cotton fibers can be used as natural fibers and e.g. rayon can be used as synthetic fibers. Also said covering layer 2 is generally formed of appropriately thick layer of non-woven fibers, which are however determined in such a manner that liquid is allowed to pass through said layer of fibers and that in particular the best possible sliding ability is achieved, namely the lowest possible coefficient of friction on the outer surface of the tampon.

The invention provides that the covering layer 2 consists of thermoplastic fibers, in particular of two-component fibers, which include at least one outwardly located thermoplastic component, or e.g. of a mixture of one-component fibers, where at least a portion of fibers represent thermoplastic fibers. Regarding the tampon according to Fig. 1, two-component fibers are used, where the core consisting of polyester (PES) is surrounded with a thermoplastic wrapper consisting of high-density polyethylene (PE), wherein the ratio is PE : PES = 34% : 66% up to PE : PES = 50% : 50%, and wherein a specific density of such fabric per surface unit is 12 to 20 g/m² and preferably at least approximately 16 g/m².

The tampon is manufactured in such a manner that in the first stage appropriately thick layer of said fabric is prepared for the purposes of forming a core 1, by means of which then a strip 10 is formed, which has appropriate length and width is formed. The width of said strip 10 is adapted to the length of the tampon, while the length of said strip is determined in accordance with each desired diameter of a blank for manufacturing a tampon, and consequently to each expected diameter of each tampon as such.

Upon that, said strip 10 is equipped with a not shown string and also with a strip 20, which is used for the purposes of forming a covering layer 2 of the tampon, and wound around its transversal axis into appropriate roller, by which approximately cylindrical blank 100 is obtained, the length of which at least approximately corresponds to the width of said strip 10 and which is then pressed in its radial direction or in at least approximately radial direction by means of appropriately shaped jaws 31, 32, which are equidistantly arranged in the circumferential direction of said blank 100, and by means of appropriate machine 3, which is known to those skilled in the art and can be e.g. one of commercially available machines manufactured by Swiss company Ruggli AG, by which a tampon is obtained which is on the one end portion formed with a narrowed and rounded tip and on the other end portion equipped with a not shown string, which makes removing the tampon after the use thereof much easier. The shape of such manufactured tampon, which is commercially available and is at present widely used, is known among others also from the patent literature and is of that reason here neither precisely explained in the description nor presented in the drawing.

Thanks to said radial compression, such manufactured tampon (Fig. 1) comprises a plurality of grooves 11', 12', 13', 14', 15', 16', 17', 18', which extend in the longitudinal direction thereof and are equidistantly arranged in the circumferential direction thereof, where a rib 11", 12", 13", 14", 15", 16", 17", 18" having a slightly rounded convex outer surface 110", 120", 130", 140", 150", 160", 170", 180" is available between each two neighboring grooves 11', 12', 13', 14', 15', 16', 17', 18'. In the shown embodiment of the tampon according to Fig. 1, eight grooves 11', 12', 13', 14', 15', 16', 17', 18' and eight a ribs 11", 12", 13", 14", 15", 16", 17", 18" are available on the surface of the tampon, wherein each neighboring ribs 11", 12", 13", 14", 15", 16", 17", 18" may also abut each other in the area of their side flanks.

The absorbent core 1 is essentially compressed in the central area 19 of the tampon. In this, those skilled in the art should understand that the number of grooves 11', 12', 13', 14', 15', 16', 17', 18' may also be different and is generally between 2 and 10, or optionally even more.

As mentioned, said absorbent core 1 is surrounded with a covering layer 2 around the complete circumference of the tampon. This means that said covering layer 2 extends continuously and without interruptions along the complete circumference of the tampon and is present both within said grooves 11', 12', 13', 14', 15', 16', 17', 18' and also in the area of said ribs 11", 12", 13", 14", 15", 16", 17", 18" including with convex outer surfaces 110", 120", 130", 140", 150", 160", 170", 180" thereof, which together form at least approximately cylindrical outer surface of the tampon. Previously described fibrous material as such, although not specially smoothed or otherwise treated, generally meets the requirements of commonly expected sliding ability of the outer surface of the tampon, by which the coefficient of friction is still rather high, but at the same time its permeability for liquids is also perfect.

In order to ensure essentially improved sliding ability and correspondingly low coefficient of friction on the outer surface of the tampon, during the manufacturing process by means of the radial compression of the blank 100 by means of jaws 31, 32 (Fig. 3), by which a tampon is obtained with a core 1, grooves 11', 12', 13', 14', 15', 16', 17', 18' and ribs 11", 12", 13", 14", 15", 16", 17", 18" with convex outer surfaces 110", 120", 130", 140", 150", 160", 170", 180" and being surrounded with a covering layer 2 around its complete circumference, such tampon is promptly upon performing said radial compression transferred throughout a conical passage 330 of appropriately heated die 33. Depending on each desired composition or combination of fibers of the covering layer 2, said die 33 is heated in the shown embodiment according to Fig. 3 by means of electric heating means 34 in such extent that the temperature on the inner surface of said conical passage 330 is 120°C to 180°C, and preferably from 122°C to 148°C.

When a previously formed tampon is e.g. by means of a pin 35 transferred throughout a conical passage 330 of appropriately heated die 33, only the convex outer surfaces 110", 120", 130", 140", 150", 160", 170", 180" of the ribs 11", 12", 13", 14", 15", 16", 17", 18" are brought into contact with the surface of the passage 330 of the heated die 33. Thanks to said composition of the fibrous material in the covering layer 2 of the tampon, where the fibers consist of a core of PE and the wrapper of PES, or the fibers of PES and fibers of PE are mixed, during such treatment of the tampon in accordance with the invention at the temperature between 122°C and 148°C, or generally between 120°C and 180°C said of PE fibers or PE component of fibers are then plasticized. Since in such state the tampon is also transferred throughout said conical passage 330 of the heated die 33, the tampon is exposed to slight and uniform compression in the area of the outer convex surfaces 110", 120", 130", 140", 150", 160", 170", 180" of the ribs 11", 12", 13", 14", 15", 16", 17", 18", which is acting towards the surface of the covering layer 2 i.e. radially inwards with respect to the tampon as such, which in combination with previously indicated plasticizing results in smoothing of the covering layer 2 solely in the area of said convex outer surfaces 110", 120", 130", 140", 150", 160", 170", 180" of the ribs 11", 12", 13", 14", 15", 16", 17", 18" but not also in the area of side flanks of the ribs 11", 12", 13", 14", 15", 16", 17", 18" and grooves 11', 12', 13', 14', 15', 16', 17', 18', where the structure of said covering layer 2 remains completely unchanged, by which in the lastly mentioned areas also the permeability for liquids and capability of transferring the liquid towards the absorbent core 1 remains completely unchanged.

## Claims

1. Hygienic tampon having improved sliding ability on its outer surface, such tampon comprising an absorbent core (1) consisting of absorbent natural or artificial fibers, as well as a of a continuous covering layer (2), which contains thermoplastic fibers which are approximately uniformly distributed across the surface thereof and by which said core (1) is surrounded around its complete circumferential area, wherein the first end portion of such tampon is optionally narrowed and formed with a slightly rounded tip, while the second end portion thereof is equipped with a string intended for easier removing the tampon after the use, and wherein such tampon is due to at least approximately radial compression on its surface provided with appropriate number e.g. at least two to ten radial grooves (11', 12', 13', 14', 15', 16', 17', 18'), which extend at least approximately parallel to each other in the longitudinal direction of the tampon or are inclined at certain angle with respect to said longitudinal direction and which are equidistantly apart from each other distributed in the circumferential direction of the tampon, so that between each two neighboring grooves (11', 12', 13', 14', 15', 16', 17', 18') there is a rib (11", 12", 13", 14", 15", 16", 17", 18") having a slightly rounded convex outer surface (110", 120", 130", 140", 150", 160", 170", 180"), so that such outer surfaces (110", 120", 130", 140", 150", 160", 170", 180") of all ribs (11", 12", 13", 14", 15", 16", 17", 18") together form at least approximately cylindrical outer surface of the tampon, **characterized in that** the tampon is smoothed exclusively in the area of convex outer surfaces (110", 120", 130", 140", 150", 160", 170", 180") of each available ribs (11", 12", 13", 14", 15", 16", 17", 18"), by which on said outer surface of the tampon the friction coefficient is reduced and the sliding ability is correspondingly improved,
wherein the covering layer (2) consists either of two-component fibers, where at least the outer component thereof consists of a thermoplastic material, or of a mixture of fibers, where at least a portion of these fibers represent thermoplastic fibers, and is at least in the area of convex outer surfaces (110", 120", 130", 140", 150", 160", 170", 180") of the ribs (11", 12", 13", 14", 15", 16", 17", 18") smoothed by heating said thermoplastic fibers or a thermoplastic component of said fibers up to the temperature of plasticizing of the thermoplastic material of fibers by simultaneously establishing a smooth uniform compression towards the outer surface of the covering layer (2) in the radial direction regarding the tampon by means of transferring thereof throughout a conical passage (330) of appropriately heated die (33) such that only the convex outer surfaces (110", 120", 130", 140", 150", 160", 170", 180") of the ribs (11", 12", 13", 14", 15", 16", 17", 18") are brought into contact with the surface of said passage (33) of the heated die (3), and wherein the covering layer (2) consists of two-component fibers, which are formed with a core on the basis of polyester (PES) and with a wrapper on the basis of high density polyethylene (PE) or optionally of a mixture of fibers of the same content, where the ratio PE : PES = 34% : 66% up to PE : PES = 50% : 50%, and where the specific surface density of such two-component fibers is 12 to 20 g/m² and preferably around 16 g/m², wherein said covering layer (2) is smoothed in the area of convex outer surfaces (110", 120", 130", 140", 150", 160", 170", 180") of the ribs (11", 12", 13", 14", 15", 16", 17", 18") by means of plasticizing said polyethylene (PE) component of fibers at the temperature within the range between 120°C and 180°C, preferably between 122°C and 148°C, by simultaneously exposing such plasticized fibers on the outer surface of the covering layer (2) to a smooth uniform compression acting inwardly in the radial direction regarding the tampon.

2. Process of manufacturing a hygienic tampon having improved sliding ability on its outer surface, such process comprising steps of
- preparing of appropriately thick layer of a non-woven fabric on the basis of natural or synthetic fibers for the purposes of forming an absorbent core (1);
- preparing of appropriately thick layer of a non-woven fabric for the purposes of forming a covering layer (2) of the tampon, in which the content of fibers is determined in such manner that fibers are either two-component fibers in which one component consists of a thermoplastic material, or at least a portion of fibers are thermoplastic fibers;
- cutting said fabric into a strip (10) intended to form said absorbent core (1) and having a width adjusted to the length of the tampon and the length adjusted to each desired diameter of the tampon;
- cutting said fabric into a strip intended to form said covering layer (2) and having a width adjusted to the length of the tampon and the length adjusted to each desired diameter or circumference of the tampon;
- assembling said strip (10) intended to form the absorbent core (1) and said strip (20) intended to form the covering layer (2) by means of overlapping said strip (20) of the covering layer (2) over a pre-determined area or the strip (10) of the core (1), wherein this step also includes appropriate placing a string, which is intended for removing the tampon after the use thereof;
- winding said strip (10) intended to form a core (1) around the transversal axis of the strip, by which at least approximately cylindrical blank (100) is formed, which is across the complete circumferential area or at least across the majority of said circumferential area surrounded by continuously extending strip (20) intended to form the covering layer (2), wherein the diameter of such blank (100) is adjusted by taking into consideration each expected diameter of the tampon, and wherein the length of such blank (100) is adjusted with respect to each expected length of the tampon;
- introducing said blank (100) into appropriate press in order to perform a radial compression thereof by means of appropriate jaws (31, 32) and to obtain at least cylindrical tampon having a narrowed and slightly rounded tip on its one end portion and a desired number, which corresponds to the number of jaws (31, 32), of radial grooves (11', 12', 13', 14', 15', 16', 17', 18'), which are pressed in the circumferential surface of the tampon and extend at least essentially parallel to each other either in the longitudinal direction of the tampon or are inclined with respect to said longitudinal direction, and which are equidistantly spaced from each other in the circumferential direction of the tampon, so that a rib (11", 12", 13", 14", 15", 16", 17", 18") is formed between each two neighboring grooves (11', 12', 13', 14', 15', 16', 17', 18'), which is equipped with a slightly rounded convex outer surface (110", 120", 130", 140", 150", 160", 170", 180"), so that convex surfaces (110", 120", 130", 140", 150", 160", 170", 180") of all ribs (11", 12", 13", 14", 15", 16", 17", 18") form an essentially cylindrical outer surface of the tampon; and
- smoothing said outer surface of the tampon exclusively in the area of said convex outer surfaces (110", 120", 130", 140", 150", 160", 170", 180") of the ribs (11", 12". 13", 14", 15", 16", 17", 18") by means of pushing such formed tampon throughout a conical passage (33) of a die (3) in the axial direction thereof, by which the tampon is exposed to a slight compression acting radially inwards, **characterized in that** said step of smoothing the outer surface of the tampon is performed as smoothing of the outer surface of the covering layer (2) consisting either at least partially of thermoplastic fibers, which are uniformly distributed across the surface area thereof, or of two-component fibers comprising outwardly located component on the basis of thermoplastic material, wherein exclusively in the area of said convex outer surfaces (110", 120", 130", 140", 150", 160", 170", 180") of the ribs (11", 12", 13", 14", 15", 16", 17", 18") on the tampon said thermoplastic fibers or a thermoplastic component of fibers are/is heated on the temperature of plasticizing of said thermoplastic fibers or said thermoplastic component of fibers, upon which such plasticized fibers are exposed to a slight and uniform compression, which is acting radially inwards with respect to the tampon such that only the convex outer surfaces (110", 120", 130", 140", 150", 160", 170", 180") of the ribs (11", 12", 13", 14", 15", 16", 17", 18") are brought into contact with the surface of said passage (33) of the heated die (3),
wherein the covering layer (2), which consists of fibers having a core of polyester (PES) wrapped by high density polyethylene (PE) component, wherein the ratio PE : PES = 34% : 66% up to PE : PES = 50% : 50%, or optionally of two-component mixture of fibers of the same content of said materials, and wherein the surface density of such two-component fabric is 12 to 20 g/m² and preferably around 16 g/m², is smoothed in the area of convex outer surfaces (110", 120", 130", 140", 150", 160", 170", 180") of the ribs (11", 12", 13", 14", 15", 16", 17", 18") by means of heating said thermoplastic fibers up to the temperature of plasticizing said polyethylene (PE) fibers or a polyethylene (PE) component of said fibers by simultaneously exposing such plasticized fibers on the outer surface of the covering layer (2) to a slight and uniform compression, which is acting inwardly in the radial direction regarding the tampon,
and wherein for the purposes of plasticizing of the polyethylene (PE) component, said fibers are heated up to the temperature within the range between 120°C and 180°C, preferably between 122°C and 148°C, upon which such plasticized fibers are exposed to a slight uniform compression, which is acting inwardly in the radial direction regarding the tampon.

## Patentansprüche

1. Hygienetampon mit einer verbesserten Gleitfähigkeit an seiner Außenfläche, wobei ein solcher Tampon einen Saugkern (1) umfasst, der aus absorbierenden Natur- oder Kunstfasern sowie aus einer durchgehenden Deckschicht (2) besteht, die thermoplastische Fasern enthält, die in etwa gleichmäßig über dessen Oberfläche verteilt sind und von denen der Kern (1) um seinen gesamten Umfangsbereich umgeben ist, wobei der erste Endabschnitt eines solchen Tampons gegebenenfalls verengt und mit einer leicht abgerundeten Spitze geformt ist, während der zweite Endabschnitt von diesem mit einem Faden versehen ist, der dafür vorgesehen ist, den Tampon nach Verwendung leichter zu entfernen, und wobei ein solcher Tampon durch eine zumindest in etwa radiale Pressung auf seiner Oberfläche mit einer geeigneten Anzahl von z. B. wenigstens zwei bis zehn radialen Nuten (11', 12', 13', 14', 15', 16', 17', 18') versehen ist, die sich zumindest in etwa parallel zueinander in der Längsrichtung des Tampons erstrecken oder in einem bestimmten Winkel relativ zu der Längsrichtung geneigt sind und die abstandsgetreu voneinander getrennt in der Umfangsrichtung des Tampons verteilt sind, so dass zwischen jeweils zwei benachbarten Nuten (11', 12', 13', 14', 15'; 16', 17', 18') eine Rippe (11", 12", 13", 14", 15", 16"; 17", 18") vorhanden ist, die eine leicht abgerundete konvexe Außenfläche (110", 120", 130", 140", 150", 160", 170", 180") aufweist; so dass solche Außenflächen (110", 120", 130", 140", 150", 160", 170", 180") aller Rippen (11", 12", 13", 14", 15", 16", 17", 18") zusammen eine zumindest in etwa zylindrische Außenfläche des Tampons bilden, **dadurch gekennzeichnet, dass** der Tampon ausschließlich im Bereich von konvexen Außenflächen (110", 120", 130", 140", 150", 160", 170", 180") aller verfügbaren Rippen (11", 12", 13", 14", 15", 16", 17", 18") geglättet ist, wodurch auf der Außenfläche des Tampons der Reibungskoeffizient verringert und die Gleitfähigkeit entsprechend verbessert ist,
wobei die Deckschicht (2) entweder aus Zwei-Komponenten-Fasern, wobei zumindest die äußere Komponente von diesen aus einem thermoplastischen Material besteht, oder aus einem Gemisch aus Fasern besteht, wobei zumindest ein Teil dieser Fasern thermoplastische Fasern sind, und diese zumindest im Bereich von konvexen Außenflächen (110", 120", 130", 140", 150", 160", 170", 180") der Rippen (11", 12", 13", 14", 15", 16", 17", 18") geglättet ist, indem die thermoplastischen Fasern oder eine thermoplastische Komponente der Fasern bis zu einer Weichmachungstemperatur des thermoplastischen Materials von Fasern erwärmt wird/werden, wobei gleichzeitig eine glatte gleichmäßige Pressung in Richtung zur Außenfläche der Deckschicht (2) in radialer Richtung in Bezug auf den Tampon durch Führen von diesem komplett durch einen konischen Durchgang (330) einer auf geeignete Weise erwärmten Düse (33) erfolgt, so dass nur die konvexen Außenflächen (110", 120", 130", 140", 150", 160", 170", 180") der Rippen (11 ", 12", 13", 14", 15", 16", 17", 18") mit der Oberfläche des Durchgangs (33) der erwärmten Düse (3) in Kontakt gebracht werden,
und wobei die Deckschicht (2) aus Zwei-Komponenten-Fasern besteht, die mit einem Kern auf Basis von Polyester (PES) und mit einer Hülle auf Basis von Polyethylen (PE) hoher Dichte gebildet sind, oder die gegebenenfalls aus einem Gemisch aus Fasern mit dem gleichen Gehalt besteht, wobei das Verhältnis PE : PES = 34% : 66% bis zu PE : PES = 50% : 50% beträgt und wobei die spezifische Oberflächendichte solcher Zwei-Komponenten-Fasern 12 bis 20 g/m² und vorzugsweise etwa 16 g/m² beträgt, wobei die Deckschicht (2) im Bereich von konvexen Außenflächen (110", 120", 130", 140", 150", 160", 170", 180") der Rippen (11", 12", 13", 14", 15", 16", 17", 18") durch Weichmachen der Polyethylen-(PE)-Komponente von Fasern bei der Temperatur im Bereich zwischen 120 °C und 180 °C, vorzugsweise zwischen 122 °C und 148 °C, geglättet wird, wobei solche weichgemachten Fasern gleichzeitig auf der Außenfläche der Deckschicht (2) einer glatten gleichmäßigen Pressung unterworfen werden, die in radialer Richtung in Bezug auf den Tampon nach innen wirkt.

2. Verfahren zur Herstellung eines Hygienetampons mit einer verbesserten Gleitfähigkeit an seiner Außenfläche, wobei ein solches Verfahren die folgenden Schritte umfasst:
- Vorbereiten einer geeignet dicken Schicht eines Vliesstoffes auf Basis von Natur- oder Synthesefasern zum Zweck des Bildens eines Saugkerns (1);
- Vorbereiten einer geeignet dicken Schicht eines Vliesstoffes zum Zweck des Bildens einer Deckschicht (2) des Tampons, bei der der Gehalt an Fasern derart bestimmt wird, dass die Fasern entweder Zwei-Komponenten-Fasern sind, bei denen eine Komponente aus einem thermoplastischen Material besteht, oder zumindest ein Teil der Fasern thermoplastische Fasern sind;
- Schneiden des Stoffs in einen Streifen (10) zum Bilden des Saugkerns (1) mit einer Breite, die auf die Länge des Tampons eingestellt ist, und wobei die Länge auf jeden gewünschten Durchmesser des Tampons eingestellt wird;
- Schneiden des Stoffs in einen Streifen zum Bilden der Deckschicht (2) mit einer Breite, die auf die Länge des Tampons eingestellt wird, und wobei die Länge auf jeden gewünschten Durchmesser oder Umgang des Tampons eingestellt wird;
- Zusammensetzen des Streifens (10), der den Saugkern (1) bilden soll, und des Streifens (20), der die Deckschicht (2) bilden soll, durch Überlagern des Streifens (20) der Deckschicht (2) über einen vorbestimmten Bereich des Streifens (10) des Kerns (1), wobei dieser Schritt auch das geeignete Positionieren eines Fadens umfasst, der zum Entfernen des Tampons nach dessen Verwendung vorgesehen ist;
- Wickeln des Streifens (10), der einen Kern (1) bilden soll, um die Querachse des Streifens, wodurch ein zumindest in etwa zylindrischer Rohling (100) gebildet wird, welcher über den gesamten Umfangsbereich oder zumindest über den Großteil des Umfangsbereich von dem sich durchgehend erstreckenden Streifen (20) umgeben ist, der die Deckschicht (2) bilden soll, wobei der Durchmesser eines solchen Rohlings (100) eingestellt wird, indem jeder erwartete Durchmesser des Tampons in Betracht gezogen wird, und wobei die Länge eines solchen Rohlings (100) in Bezug auf jede erwartete Länge des Tampons eingestellt wird;
- Einführen des Rohlings (100) in eine geeignete Presse, um durch geeignete Klemmbacken (31, 32) eine radiale Pressung an diesem vorzunehmen und um einen zumindest zylindrischen Tampon mit einer verengten und leicht abgerundeten Spitze an seinem einen Endabschnitt und einer gewünschten Anzahl, die der Anzahl an Klemmbacken (31, 32) entspricht, an radialen Nuten (11', 12', 13', 14', 15', 16', 17', 18') zu erhalten, welche in die Umfangsfläche des Tampons gepresst werden und sich zumindest im Wesentlichen parallel zueinander in der Längsrichtung des Tampons erstrecken oder in Bezug auf diese Längsrichtung geneigt sind, und welche in Umtangsrichtung des Tampons abstandsgetreu voneinander beabstandet sind, so dass eine Rippe (11", 12", 13", 14", 15", 16", 17", 18") zwischen jeweils zwei benachbarten Nuten (11', 12', 13', 14', 15', 16', 17', 18') gebildet ist, die mit einer leicht abgerundeten konvexen Außenfläche (110", 120", 130", 140", 150", 160", 170", 180") versehen ist, so dass konvexe Oberflächen (110", 120", 130", 140", 150", 160", 170", 180") aller Rippen (11 ", 12", 13", 14", 15", 16", 17", 18") eine im Wesentlichen zylindrische Außenfläche des Tampons bilden; und
- Glätten der Außenfläche des Tampons nur im Bereich der konvexen Außenflächen (110", 120" 130", 140", 150", 160", 170", 180") der Rippen (11 ", 12", 13", 14", 15", 16", 17", 18") durch Drücken eines auf diese Weise geformten Tampons komplett durch einen konischen Durchgang (33) einer Düse (3) in dessen axialer Richtung, wodurch der Tampon einer leichten Pressung, die radial nach innen wirkt, ausgesetzt ist, **dadurch gekennzeichnet, dass** der Schritt des Glättens der Außenfläche des Tampons als Glätten der Außenfläche der Deckschicht (2) erfolgt, die entweder zumindest teilweise aus thermoplastischen Fasern, die gleichmäßig über deren Oberflächenbereich verteilt sind, oder aus Zwei-Komponenten-Fasern besteht, die eine außen angeordnete Komponente auf Basis von thermoplastischem Material umfassen, wobei nur in dem Bereich der konvexen Außenflächen (110", 120", 130", 140", 150", 160", 170", 180") der Rippen (11", 12", 13", 14", 15", 16", 17", 18") auf dem Tampon die thermoplastischen Fasern oder eine thermoplastische Komponente der Fasern auf die Weichmachungstemperatur der thermoplastischen Fasern oder der thermoplastischen Komponente der Fasern erwärmt wird/werden, woraufhin solche weichgemachten Fasern einer leichten und gleichmäßigen Pressung unterworfen werden, die in Bezug auf den Tampon radial nach innen wirkt, so dass nur die konvexen Außenflächen (110", 120", 130", 140", 150", 160", 170", 180") der Rippen (11", 12", 13", 14", 15", 16", 17", 18") mit der Oberfläche des Durchgangs (33) der erwärmten Düse (3) in Kontakt kommen,
wobei die Deckschicht (2), die aus Fasern mit einem Kern aus Polyester (PES) besteht, der von einer Komponente von Polyethylen (PE) hoher Dichte umhüllt ist, wobei das Verhältnis von PE : PES = 34% : 66% bis zu PE : PES = 50% : 50% beträgt, oder gegebenenfalls aus einem Zwei-Komponenten-Gemisch von Fasern mit dem gleichen Gehalt der Materialien besteht, und wobei die Oberflächendichte eines solchen Zwei-Komponentenstoffs 12 bis 20 g/m² und vorzugsweise etwa 16 g/m² beträgt, in dem Bereich der konvexen Außenflächen (110", 120", 130", 140", 150", 160", 170", 180") der Rippen (11", 12", 13", 14", 15", 16", 17", 18") durch Erwärmung der thermoplastischen Fasern bis zur Weichmachungstemperatur der Polyethylen-(PE)-Fasern oder einer Polyethylen-Komponente der Fasern geglättet wird, indem solche weichgemachten Fasern auf der Außenfläche der Deckschicht (2) gleichzeitig einer leichten und gleichmäßigen Pressung unterworfen werden, die in Bezug auf den Tampon in radialer Richtung nach innen wirkt,
und wobei zum Zweck des Weichmachens der Polyethylen-(PE)-Komponente die Fasern bis zu einer Temperatur im Bereich zwischen 120°C und 180°C, vorzugsweise zwischen 122°C und 148°C erwärmt werden, woraufhin solche weichgemachten Fasern einer leichten gleichmäßigen Pressung ausgesetzt werden, die in radialer Richtung in Bezug auf den Tampon nach innen wirkt.

## Revendications

1. Tampon hygiénique possédant une capacité de glissement perfectionnée sur sa surface externe, ledit tampon comprenant une partie centrale absorbante (1) constituée par des fibres absorbantes naturelles ou synthétiques, et par une couche de revêtement continue (2) qui contient des fibres thermoplastiques qui sont distribuées de manière approximativement uniforme sur toute sa surface et qui entourent ladite partie centrale (1) le long de toute la zone circonférentielle de cette dernière, dans lequel de manière facultative la première portion terminale dudit tampon est rétrécie et configurée avec une extrémité légèrement arrondie, tandis que sa deuxième portion terminale est équipée d'une ficelle pour retirer plus aisément le tampon après son utilisation, et dans lequel ledit tampon est muni, via une compression s'exerçant au moins de manière approximative en direction radiale sur sa surface, d'un nombre approprié, par exemple d'au moins deux à dix rainures radiales (11', 12', 13', 14', 15', 16', 17', 18'), qui s'étendent au moins de manière approximative parallèlement les unes aux autres dans la direction longitudinale du tampon ou qui sont inclinées en formant un certain angle par rapport à ladite direction longitudinale, et qui sont distribuées en étant espacées de manière réciproquement équidistante dans la direction circonférentielle du tampon, de manière telle qu'entre deux rainures adjacentes respectives (11', 12', 13', 14', 15', 16'. 17', 18'), on prévoit une nervure (11", 12", 13", 14", 15", 16", 17", 18") possédant une surface externe convexe légèrement arrondie (110", 120", 130", 140", 150", 160", 170", 180"), tant et si bien que les surfaces externes (110", 120", 130", 140", 150", 160", 170", 180") de toutes les nervures (11", 12", 13", 14", 15", 16", 17", 18") forment ensemble la surface externe au moins approximativement cylindrique du tampon, **caractérisé en ce que** le tampon est rendu lisse à titre exclusif dans la zone des surfaces externes convexes (110", 120", 130", 140", 150", 160", 170", 180") de chaque nervure disponible (11", 12", 13", 14", 15", 16", 17", 18"), ce qui permet de réduire le coefficient de friction sur ladite surface externe du tampon, donnant lieu à une amélioration correspondante de la capacité de glissement ; dans lequel la couche de revêtement (2) est constituée, soit par des fibres à deux composants, au moins leur composant externe étant alors constitué d'une matière thermoplastique, soit par un mélange de fibres, au moins une portion de ces fibres représentant alors des fibres thermoplastiques, et étant rendue lisse, au moins dans la zone des surfaces externes convexes (110", 120", 130", 140", 150", 160", 170", 180") des nervures (11", 12", 13", 14", 15", 16", 17", 18"), via un chauffage desdites fibres thermoplastiques ou d'un composant thermoplastique desdites fibres jusqu'à la température de plastification de la matière thermoplastique des fibres en exerçant de manière simultanée une compression régulière et uniforme en direction de la surface externe de la couche de revêtement (2) dans la direction radiale par rapport au tampon via son transfert à travers un passage conique (330) d'une filière chauffée (33) de manière appropriée de manière telle que uniquement les surfaces externes convexes (110", 120", 130", 140", 150", 160", 170", 180") des nervures (11", 12", 13", 14", 15", 16", 17", 18") entrent en contact avec la surface dudit passage (33) de la filière chauffée (3) ; et dans lequel la couche de revêtement (2) est constituée par des fibres à deux composants qui sont configurées pour comprendre une partie centrale à base de polyester (PES) et une enveloppe à base de polyéthylène haute densité (PE) ou de manière facultative par un mélange de fibres possédant la même teneur, le rapport PE : PES s'élevant de PE : PES = 34 % : 66 % à PE : PES = 50 % : 50 %, et dans lequel la densité surfacique spécifique desdites fibres à deux composants s'élève de 12 à 20 g/m² et de préférence à environ 16 g/m², dans lequel ladite couche de revêtement (2) est rendue lisse dans la zone des surfaces externes convexes (110", 120", 130", 140", 150", 160", 170", 180") des nervures (11", 12", 13", 14", 15", 16", 17", 18") au moyen d'une plastification dudit composant de polyéthylène (PE) des fibres à une température qui se situe dans la plage entre 120 °C et 180 °C, de préférence entre 122 °C et 148 °C, en exposant de manière simultanée lesdites fibres plastifiées sur la surface externe de la couche de revêtement (2) à une compression uniforme et régulière qui s'exerce vers l'intérieur dans la direction radiale par rapport au tampon.

2. Procédé de fabrication d'un tampon hygiénique possédant une capacité de glissement perfectionnée sur sa surface externe, ledit procédé comprenant les étapes dans lesquelles :
- on prépare une couche d'épaisseur appropriée d'une étoffe non tissée à base de fibres naturelles ou synthétiques dans le but d'obtenir une partie centrale absorbante (1) ;
- on prépare une couche d'épaisseur appropriée d'une étoffe non tissée dans le but d'obtenir une couche de revêtement (2) du tampon, la teneur en fibres étant déterminée de manière telle que, soit les fibres représentent des fibres à deux composants dans lesquelles un composant est constitué d'une matière thermoplastique, soit au moins une portion des fibres représentent des fibres thermoplastiques;
- on découpe ladite étoffe pour obtenir une bande (10) destinée à former ladite partie centrale absorbante (1) et possédant une largeur adaptée à la longueur du tampon et une longueur adaptée à chaque diamètre désiré du tampon ;
- on découpe ladite étoffe pour obtenir une bande destinée à former ladite couche de revêtement (2) et possédant une largeur adaptée à la longueur du tampon et une longueur adaptée à chaque diamètre désiré ou à chaque circonférence désirée du tampon ;
- on assemble ladite bande (10) destinée à former la partie centrale absorbante (1) et ladite bande (20) destinée à former la couche de revêtement (2) en superposant ladite bande (20) de la couche de revêtement (2) sur une zone prédéterminée de la bande (10) de la partie centrale (1), cette étape englobant également le placement approprié d'une ficelle qui est destinée au retrait du tampon après l'utilisation de ce dernier ;
- on enroule ladite bande (10) destinée à former la partie centrale (1) autour de l'axe transversal de la bande, de manière telle que l'on obtient une ébauche au moins approximativement cylindrique (100), qui est entourée, sur toute la zone circonférentielle ou sur au moins la majeure partie de ladite zone circonférentielle, par une bande (20) s'étendant en continu, destinée à former la couche de revêtement (2), le diamètre de ladite ébauche (100) étant réglé en prenant en compte chaque diamètre escompté du tampon, et la longueur de ladite ébauche (100) étant réglée par rapport à chaque longueur escomptée du tampon ;
- on introduit ladite ébauche (100) dans une presse appropriée pour mettre en oeuvre sa compression à radiale au moyen de mâchoires appropriées (31, 32) et pour obtenir un tampon au moins cylindrique possédant une extrémité rétrécie et légèrement arrondie à une de ses portions terminales et un nombre désiré, qui correspond au nombre de mâchoires (31, 32), de rainures radiales (11', 12'. 13', 14', 15', 16', 17', 18'), qui sont comprimées dans la surface circonférentielle du tampon et qui, soit s'étendent au moins de manière essentielle parallèlement les unes aux autres dans la direction longitudinale du tampon, soit sont inclinées par rapport à ladite direction longitudinale, et qui sont espacées de manière réciproquement équidistante dans la direction circonférentielle du tampon, si bien que l'on obtient, entre deux rainures adjacentes respectives (11', 12', 13', 14', 15', 16', 17', 18'), la formation d'une nervure (11", 12", 13", 14", 15", 16", 17", 18") qui est munie d'une surface externe convexe légèrement arrondie (110", 120", 130", 140", 150", 160", 170", 180"), si bien que les surfaces convexes (110", 120", 130", 140", 150", 160", 170", 180") de toutes les nervures (11", 12", 13", 14", 15", 16", 17", 18") forment une surface externe essentiellement cylindrique du tampon ; et
- on rend lisse ladite surface externe du tampon, à titre exclusif dans la zone desdites surfaces externes convexes (110", 120", 130", 140", 150", 160", 170", 180") des nervures (11", 12", 13", 14", 15", 16", 17", 18") en poussant ledit tampon ainsi obtenu à travers un passage conique (33) d'une filière (3) dans la direction axiale de cette dernière, de manière telle que le tampon est exposé à une légère compression qui s'exerce vers l'intérieur en direction radiale, **caractérisé en ce que** ladite étape, par laquelle on rend lisse la surface externe du tampon, est mise en oeuvre en rendant lisse la surface externe de la couche de revêtement (2) constituée au moins en partie, soit par des fibres thermoplastiques qui sont distribuées de manière uniforme sur toute sa zone superficielle, soit par des fibres à deux composants comprenant un composant disposé à l'extérieur à base d'une matière thermoplastique, dans lequel, exclusivement dans la zone desdites surfaces externes convexes (110", 120", 130", 140", 150", 160", 170", 180") des nervures (11", 12", 14", 15", 16", 17", 18") sur le tampon, on chauffe lesdites fibres thermoplastiques ou ledit composant thermoplastique des fibres à la température de plastification desdites fibres thermoplastiques ou dudit composant thermoplastique des fibres ; après quoi, on expose lesdites fibres plastifiées à une compression légère et uniforme, qui s'exerce vers l'intérieur en direction radiale par rapport au tampon, de manière telle que uniquement les surfaces externes convexes (110", 120", 130", 140", 150", 160", 170", 180") des nervures (11", 12", 13", 15", 16", 17", 18") entrent en contact avec la surface dudit passage (33) de la filière chauffée (3) ;
dans lequel la couche de revêtement (2), qui est constituée par des fibres possédant une partie centrale de polyester (PES) enveloppée d'un composant de polyéthylène haute densité (PE), le rapport PE : PES s'élevant de PE : PES = 34 % : 66 % à PE : PES = 50 % : 50 %, ou de manière facultative par un mélange de fibres à deux composants possédant la même teneur matérielle, et dans lequel la densité surfacique de ladite étoffe à deux composants s'élève de 12 à 20 g/m² et de préférence à environ 16 g/m², est rendue lisse dans la zone des surfaces externes convexes (110", 120", 130", 140", 150", 160", 170", 180") des nervures (11", 12", 13", 14", 15", 16", 17", 18") en chauffant lesdites fibres thermoplastiques jusqu'à la température de plastification desdites fibres de polyéthylène (PE) ou du composant de polyéthylène (PE) desdites fibres en exposant de manière simultanée lesdites fibres plastifiées sur la surface externe de la couche de revêtement (2) à une compression légère et uniforme qui s'exerce vers l'intérieur dans la direction radiale par rapport au tampon ;
et dans lequel, pour la plastification du composant de polyéthylène (PE), lesdites fibres sont chauffées jusqu'à une température qui se situe dans la plage entre 120 °C et 180 °C, de préférence entre 122 °C et 148 °C ; après quoi, lesdites fibres plastifiées sont exposées à une compression légère et uniforme qui s'exerce vers l'intérieur dans la direction radiale par rapport au tampon.
